# EUROPEAN PATENT APPLICATION

(11) **EP 3 628 683 A1**
(43) Date of publication of application: **01.04.2020**
(21) Application number: 18197755.4
(22) Date of filing: 28.09.2018
(51) Int. Cl.: C07K 14/605, A61K 38/26

(54) **FORMULATIONS OF GLUCAGON-LIKE-PEPTIDE-2 (GLP-2) ANALOGUES**

(71) Applicant: Zealand Pharma A/S, 2860 Søborg (DK)
(72) Inventor: The designation of the inventor has not yet been filed
(74) Representative: Mewburn Ellis LLP

(57) **Abstract**

Stable liquid pharmaceutical formulation of glucagon-like-peptide-2 (GLP-2) analogues are disclosed and their medical use, for example in the treatment and/or prevention of stomach and bowel-related disorders and for ameliorating side effects of chemotherapy and radiation therapy.

## Description

### Field of the Invention

The present invention relates to formulations of glucagon-like-peptide-2 (GLP-2) analogues and their medical use, for example in the treatment and/or prevention of stomach and bowel-related disorders and for ameliorating side effects of chemotherapy and radiation therapy.

### Background of the Invention

Human GLP-2 is a 33-amino-acid peptide with the following sequence: Hy-His-Ala-Asp-Gly-Ser-Phe-Ser-Asp-Glu-Met-Asn-Thr-Ile-Leu-Asp-Asn-Leu-Ala-Ala-Arg-Asp-Phe-Ile-Asn-Trp-Leu-Ile-Gln-Thr-Lys-Ile-Thr-Asp-OH. It is derived from specific post-translational processing of proglucagon in the enteroendocrine L cells of the intestine and in specific regions of the brainstem. GLP-2 binds to a single G-protein-coupled receptor belonging to the class II glucagon secretin family.

GLP-2 has been reported to induce significant growth of the small intestinal mucosal epithelium via the stimulation of stem cell proliferation in the crypts, and by inhibition of apoptosis in the villi (Drucker et al., 1996, Proc. Natl. Acad. Sci. USA 93: 7911-7916). GLP-2 also has growth effects on the colon. Furthermore, GLP-2 inhibits gastric emptying and gastric acid secretion (Wojdemann et al., 1999, J. Clin. Endocrinol. Metab. 84: 2513-2517), enhances intestinal barrier function (Benjamin et al., 2000, Gut 47: 112-119), stimulates intestinal hexose transport via the upregulation of glucose transporters (Cheeseman, 1997, Am. J. Physiol. R1965-71), and increases intestinal blood flow (Guan et al., 2003, Gastroenterology, 125: 136-147).

It has been recognised in the art that glucagon-like peptide-2 receptor analogues have therapeutic potential for the treatment of intestinal diseases. However, the native hGLP-2, a 33 amino acid gastrointestinal peptide, is not a useful in a clinical setting due to its very short half-life in humans of around 7 minutes for full length GLP-2 [1-33] and 27 minutes for truncated GLP-2 [3-33]. In large part, the short half-life is due to degradation by the enzyme dipeptidylpeptidase IV (DPP-IV). Accordingly, there have been attempts in the art to develop GLP-2 receptor agonists with better pharmacokinetic characteristics, in particular to improve the half-life of GLP-2 molecules. By way of example, GLP-2 analogues with substitutions have been suggested such as e.g. GLP-2 analogues containing Gly substitution at position 2 ([hGly2] GLP-2, teduglutide) which increases the half-life from seven minutes (native GLP-2) to about two hours. Acylation of peptide drugs with fatty acid chains has also proven beneficial for prolonging systemic circulation as well as increasing enzymatic stability without disrupting biological potency. However, while these attempts have improved the pharmacokinetics of GLP-2 analogues, and they are sometimes described in the art as "long acting", it must be kept in mind that this is in comparison to native hGLP-2 with half-lives of the order of several hours, rather than minutes. This in turn means that the GLP-2 analogues still need to be administered to patients one or more times per day.

US 5,789,379 discloses GLP-2 analogues for administration by injection. The analogues were provided as powdered peptides and mixed with phosphate buffered saline (PBS) prior to injection at pH of 7.3-7.4 with a GLP-2 concentration of 130 mg/ml. In some instances, the GLP-2/PBS composition was mixed with gelatin to provide a depot formed from a solution of 130 mg/l GLP-2 in PBS/15% gelatin. US 5,789,379 does not disclose stable aqueous liquid formulations of GLP-2 analogues and the GLP-2 analogues are generally reconstituted from powder prior to injection.

In WO 97/39031 and US 6,184,201, the GLP-2 analogue, [Gly²]GLP-2 is disclosed. Here the alanine in position 2 has been replaced with glycine to make the peptide resistant to DPP IV cleavage. As with US 5,789,379, the GLP-2 analogue was provided as a powdered peptide and mixed with saline, PBS or 5% dextrose prior to injection, optionally adding acetic acid as a solubility enhancer.

WO 02/066511 describes GLP-2 analogues having an extended half-life *in vivo* and their use as medicaments in the treatment of gastrointestinal disorders, such as inflammatory bowel diseases. The GLP-2 analogues were stored in lyophilized form and reconstituted for administration in media, for example using saline or PBS.

WO 01/41779 describes the use of h[Gly²]GLP-2 as a pre-treatment for inhibiting chemotherapy induced apoptosis and promoting cell survival. The h[Gly²]GLP-2 is delivered by subcutaneous or intravenous injection or infusion after reconstituting the analogue in PBS.

WO 2001/049314 is directed to formulations of GLP-2 peptides and analogues thereof exhibiting superior stability following storage and/or exposure to elevated temperatures. The GLP-2 compositions comprise a GLP-2 peptide or an analogue thereof, a phosphate buffer, L-histidine, and mannitol.

WO 2006/117565 describes GLP-2 analogues which comprise one of more substitutions as compared to [hGly²]GLP-2 and which improved biological activity *in vivo* and/or improved chemical stability, e.g. as assessed in *in vitro* stability assays. In particular, GLP-2 analogues are described which have substitutions at one or more of positions 8, 16, 24 and/or 28 of the wild-type GLP-2 sequence, optionally in combination with further substitutions at position 2 and one or more of positions 3, 5, 7, 10 and 11, and/or a deletion of one or more of amino acids 31 to 33. These substitutions may also be combined with the addition of a N-terminal or C-terminal stabilizing peptide sequence. The daily or twice daily administration of these GLP-2 analogues is also described. Among the molecules disclosed in WO 2006/117565 is glepaglutide (ZP1848) which has been designed to be stable in liquid formulations, and is typically administered by daily dosing using an injection pen.

It remains a problem in this area to improve the formulation of GLP-2 analogues, in particular to provide stable liquid formulations that are capable of long term storage without undue levels of physical or chemical degradation of the active monomeric form of the peptide occurring. In liquid formulations of peptide drugs, the chemical pathways that can operate include the formation of covalently linked dimers and oligomers of the peptide, reducing the amount of the active monomeric form of the peptide through the formation of these covalently linked high molecular weight oligomeric products. The law of mass action means that it is normally the case that the higher the concentration of a peptide drug in a formulation, the higher the probability of formation of covalently bonded oligomeric products.

It would also be a goal in the area of GLP-2 analogue formulation to provide formulations in which the viscosity of the formulation is controlled within a range that makes it suitable for use in delivery devices such as pre-filled syringes, infusion pumps, wearable injectors or auto-injectors.

### Summary of the Invention

Broadly, the present invention is based on studies reported in the examples that led to surprising findings relating to liquid formulations of GLP-2 analogues that make them suitable for long term storage as liquids and/or that makes them especially suitable for delivery by a drug delivery device.

In a first study, the inventors found that acetate present in the formulation that originates from the GLP-2 analogues has an effect on the viscosity of the formulation. This opens up the possibility of controlling the viscosity of the formulation by changing and/or controlling the acetate concentration. A low-range viscosity liquid formulation is useful clinically as it provides advantages in drug delivery device development and manufacturing by potentially reducing breakage, dosing failure, dosing imprecision and other malfunctions during drug product manufacture and/or patient use. Furthermore, low viscosity may allow a faster injection and/or the use of narrower bore (i.e., higher gauge) needles that in turn may reduce injection discomfort. This opens up the possibility of providing the formulations of the GLP-2 analogue in the form of a drug delivery device, such as a pre-filled syringe, an adjustable dose auto-injector, a disposable auto-injector, a wearable injector or an infusion pump, thereby providing patients with a ready-to-use formulation in a simpler, safer and more patient-friendly device. Controlling the formulation to higher viscosity could be suitable in other drug delivery devices.

In a second study, the present inventors found that during long term storage at 2-8°C of ZP1848 (glepaglutide), the formation of covalently bound oligomers is concentration dependent. However, contrary to the usual situation in which the law of mass action implies that covalent oligomer formation increases with increasing concentration of a peptide drug, the present inventors found that the concentration dependence for oligomer formation is inversely dependent on increasing concentration of the GLP-2 analogue. Without wishing to be bound by any particular theory, the present inventors believe that the reduction in the formation of covalently linked oligomers as GLP-2 analogue concentration increases is a result of the lysine tail of the GLP-2 analogue promoting the formation of self-associated structural assemblies of the native peptide that hinders the formation of covalently bound oligomers in the formulation. This means that the weakly self-associated species are capable of dissociating to release biologically active monomer after administration into a patient, rather than causing a loss of active species as happens when the covalently bound oligomers are formed.

In a third study, the present inventors found that the GLP-2 analogues used in the formulations of the present invention are not compatible with phosphate buffer commonly used in the prior art to reconstituted powdered or lyophilized GLP-2 compositions. This study found that only some buffers were compatible with formulating these GLP-2 analogues such that they were suitable for long term storage in liquid form.

Accordingly, in a first aspect, the present invention provides a stable liquid pharmaceutical formulation, the formulation comprising a glucagon-like peptide 2 (GLP-2) analogue, wherein the GLP-2 analogue is represented by the formula: 
R1-Z1-His-Gly-Glu-Gly-X5-Phe-Ser-Ser-Glu-Leu-X11-Thr-Ile-Leu-Asp-Ala-Leu-Ala-
Ala-Arg-Asp-Phe-Ile-Ala-Trp-Leu-Ile-Ala-Thr-Lys-Ile-Thr-Asp-Z2-R2
 wherein:
R¹ is hydrogen, C₁₋₄ alkyl (e.g. methyl), acetyl, formyl, benzoyl or trifluoroacetyl;
X5 is Ser or Thr;
X11 is Ala or Ser;
R² is NH₂ or OH; and
Z¹ and Z² are independently absent or a peptide sequence of 1-6 amino acid units of Lys;
or a pharmaceutically acceptable salt or derivative thereof;
wherein the formulation comprises:
   (a) the GLP-2 analogue at a concentration of about 2 mg/mL to about 30 mg/mL;
   (b) a buffer selected from the group consisting of a histidine buffer, mesylate buffer and acetate buffer, the buffer being present at a concentration of about 5 mM to about 50 mM;
   (c) a non-ionic tonicity modifier selected from the group consisting of mannitol, sucrose, glycerol and sorbitol at a concentration of about 90 mM to about 360 mM; and
   (d) arginine q.s. to provide a formulation having a pH of about 6.6 to about 7.4.

In some embodiments, the formulation contains 5% or less of the GLP-2 analogue in the form of covalently bonded oligomeric products. Alternatively or additionally, the total acetate concentration arising from the GLP-2 analogue in the formulation is less than or equal to 11% acetate per mg GLP-2 analogue. Alternatively or additionally, formation of covalently linked oligomers of the GLP-2 analogue is inversely dependent on the concentration of the GLP-2 analogue in the formulation.

The components of the formulation and their amounts provide a formulation with at least 90% content of the GLP-2 analogue and with less than 10% of chemical degradation products at storage for at least 18 months at 2-8°C.

In a further aspect, the present invention provides an article of manufacture or a kit comprising a container holding the stable pharmaceutical formulation of the present invention.

In a further aspect, the present invention provides a delivery device containing a liquid formulation comprising a GLP-2 analogue of present invention.

In a further aspect, the present invention provides a formulation of the glucagon-like peptide 2 (GLP-2) analogue of the present invention for use in therapy.

In a further aspect, the present invention provides a formulation of the glucagon-like peptide 2 (GLP-2) analogue of the present invention for use in a method for the treatment and/or prevention of a stomach and bowel-related disorder in a human patient.

In a further aspect, the present invention provides a process for producing a stable liquid pharmaceutical formulation comprising a glucagon-like peptide 2 (GLP-2) analogue, wherein the GLP-2 analogue is represented by the formula: 
R1-Z1-His-Gly-Glu-Gly-X5-Phe-Ser-Ser-Glu-Leu-X11-Thr-Ile-Leu-Asp-Ala-Leu-Ala-
Ala-Arg-Asp-Phe-Ile-Ala-Trp-Leu-Ile-Ala-Thr-Lys-Ile-Thr-Asp-Z2-R2
 wherein:
R¹ is hydrogen, C₁₋₄ alkyl (e.g. methyl), acetyl, formyl, benzoyl or trifluoroacetyl
X5 is Ser or Thr
X11 is Ala or Ser
R² is NH₂ or OH;
Z¹ and Z² are independently absent or a peptide sequence of 1-6 amino acid units of Lys;
or a pharmaceutically acceptable salt or derivative thereof;
wherein the process comprising formulating (a) the GLP-2 analogue at a concentration of about 2 mg/mL to about 30 mg/mL, (b) with a buffer selected from the group consisting of a histidine buffer, mesylate buffer and acetate buffer, the buffer being present at a concentration of about 5 mM to about 50 mM; (c) with a non-ionic tonicity modifier selected from the group consisting of mannitol, sucrose, glycerol and sorbitol, the non-ionic tonicity modifier being present at a concentration of about 90 mM to about 360 mM; and (d) with arginine q.s. to provide a formulation having a pH of about 6.6 to about 7.4;
wherein the formulation contains 5% or less of the GLP-2 analogue in the form of covalently bonded oligomeric products.

In a further aspect, the present invention provides the use of a formulation comprising a glucagon-like peptide 2 (GLP-2) analogue, wherein the GLP-2 analogue is represented by the formula: 
R1-Z1-His-Gly-Glu-Gly-X5-Phe-Ser-Ser-Glu-Leu-X11-Thr-Ile-Leu-Asp-Ala-Leu-Ala-
Ala-Arg-Asp-Phe-Ile-Ala-Trp-Leu-Ile-Ala-Thr-Lys-Ile-Thr-Asp-Z2-R2
 wherein:
R¹ is hydrogen, C₁₋₄ alkyl (e.g. methyl), acetyl, formyl, benzoyl or trifluoroacetyl X5 is Ser or Thr
X11 is Ala or Ser
R² is NH₂ or OH;
Z¹ and Z² are independently absent or a peptide sequence of 1-6 amino acid units of Lys;
or a pharmaceutically acceptable salt or derivative thereof;
for providing an liquid pharmaceutical formulation which is stable for 24 months when stored at 2-8°C, wherein the formulation comprises:
   (a) the GLP-2 analogue at a concentration of about 2 mg/mL to about 30 mg/mL;
   (b) a buffer selected from the group consisting of a histidine buffer, mesylate buffer and acetate buffer, the buffer being present at a concentration of about 5 mM to about 50 mM;
   (c) a non-ionic tonicity modifier selected from the group consisting of mannitol, sucrose, glycerol and sorbitol at a concentration of about 90 mM to about 360 mM; and
   (d) arginine q.s. to provide a formulation having a pH of about 6.6 to about 7.4.

In some embodiments, the formulation contains 5% or less of the GLP-2 analogue in the form of covalently bonded oligomeric products. Alternatively or additionally, the total acetate concentration arising from the GLP-2 analogue in the formulation is less than or equal to 11% acetate per mg GLP-2 analogue. Alternatively or additionally, formation of covalently linked oligomers of the GLP-2 analogue is inversely dependent on the concentration of the GLP-2 analogue in the formulation.

In a further aspect, the present invention relates to a stable liquid pharmaceutical formulation, the formulation comprising a glucagon-like peptide 2 (GLP-2) analogue, wherein the GLP-2 analogue is represented by the formula: 
R¹- His-Gly-Glu-Gly-X5-Phe-Ser-Ser-Glu-Leu-X11-Thr-Ile-Leu-Asp-Ala-Leu-Ala-Ala-
Arg-Asp-Phe-Ile-Ala-Trp-Leu-Ile-Ala-Thr-Lys-Ile-Thr-Asp-Z²-R²
 wherein:
R¹ is hydrogen, C₁₋₄ alkyl (e.g. methyl), acetyl, formyl, benzoyl or trifluoroacetyl;
X5 is Ser or Thr;
X11 is Ala or Ser;
R² is NH₂ or OH; and
Z² is a peptide sequence of 6 amino acid units of Lys;
or a pharmaceutically acceptable salt or derivative thereof.

In this aspect of the present invention, the glucagon-like peptide 2 (GLP-2) analogue may be used for the treatment and/or prevention of stomach and bowel-related disorders such as ulcers, digestion disorders, malabsorption syndromes, short-gut syndrome, cul-de-sac syndrome, inflammatory bowel disease, celiac sprue (for example arising from gluten induced enteropathy or celiac disease), tropical sprue, hypogammaglobulinemic sprue, enteritis, regional enteritis (Crohn's disease), ulcerative colitis, small intestine damage or short bowel syndrome (SBS). Alternatively or additionally, the glucagon-like peptide 2 (GLP-2) analogue may be used for the treatment and/or prevention of stomach and bowel-related disorders such radiation enteritis, infectious or post-infectious enteritis, or small intestinal damage due to toxic or other chemotherapeutic agents. In this case, treatment with the GLP-2 analogue may optionally be combined with one or more anti-cancer therapies, and may therefore comprise administering one or more chemotherapeutic agent(s) to the patient or treating the patient with radiation therapy.

In some embodiments of the present invention, in the above formula, X5 is Thr and/or X11 is Ala. Examples of these glucagon-like peptide 2 (GLP-2) analogues include:
ZP1848 H-HGEGTFSSELATILDALAARDFIAWLIATKITDKKKKKK-NH₂ (SEQ ID NO: 1)
ZP2949 H-HGEGTFSSELATILDALAARDFIAWLIATKITDKKK-OH (SEQ ID NO: 2);
ZP2711 H-HGEGTFSSELATILDALAARDFIAWLIATKITDKK-OH (SEQ ID NO: 3);
ZP2469 H-HGEGTFSSELATILDALAARDFIAWLIATKITDK-OH (SEQ ID NO: 4);
ZP1857 H-HGEGTFSSELATILDALAARDFIAWLIATKITD-NH₂ (SEQ ID NO: 5); or
ZP2530 H-HGEGTFSSELATILDALAARDFIAWLIATKITD-OH (SEQ ID NO: 6).

In some embodiments of the present invention, in the above formula X5 is Ser and/or X11 is Ser. Examples of these glucagon-like peptide 2 (GLP-2) analogues include:
ZP1846 H-HGEGSFSSELSTILDALAARDFIAWLIATKITDKKKKKK-NH₂ (SEQ ID NO: 7);
ZP1855 H-HGEGSFSSELSTILDALAARDFIAWLIATKITD-NH₂ (SEQ ID NO: 8); or
ZP2242 H-HGEGSFSSELSTILDALAARDFIAWLIATKITDK-OH (SEQ ID NO: 9).

Embodiments of the present invention will now be described by way of example and not limitation with reference to the accompanying figures. However, various further aspects and embodiments of the present invention will be apparent to those skilled in the art in view of the present disclosure.

"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

Unless context dictates otherwise, the descriptions and definitions of the features set out above are not limited to any particular aspect or embodiment of the invention and apply equally to all aspects and embodiments which are described.

### Brief Description of the Figures

Figure 1 shows a typical chromatogram showing the separation of the oligomers from the ZP1848 monomer.
Figure 2 shows how the viscosity (squares) and hydrodynamic radius (z-average) (circles) varied as a function of acetate concentration after manufacturing of the formulation. The data shows that above 11 % acetate the viscosity and hydrodynamic radius (z-average) start to increase.

### Detailed Description of the Invention

### Definitions

Unless specified otherwise, the following definitions are provided for specific terms, which are used in the above written description.

Throughout the description and claims the conventional one-letter and three-letter codes for natural amino acids are used. All amino acid residues in peptides of the invention are preferably of the L-configuration, However, D-configuration amino acids may also be present.

Preferred compounds of the present invention have at least one GLP-2 biological activity, in particular in causing growth of the intestine. This can be assessed in *in vivo* assays, for example as described in the examples of (e.g.) WO 2006/117565, in which the mass of the intestine, or a portion thereof is determined after a test animal has been treated or exposed to a GLP-2 analogue.

In some aspects of the present invention, the liquid formulations comprising a GLP-2 analogue have a total acetate concentration in the formulation of less than or equal to 11% acetate per mg GLP-2 analogue, and more preferably less than or equal to 10% acetate per mg GLP-2 analogue, more preferably less than or equal to 9% acetate per mg GLP-2 analogue, more preferably less than or equal to 8 % acetate per mg GLP-2 analogue, more preferably less than or equal to 7% acetate per mg GLP-2 analogue, still more preferably less than or equal to 6% acetate per mg GLP-2 analogue. Thus, for example, for a formulation having 20 mg/mL of the GLP-2 analogue, the total acetate concentration will be less than or equal to 37 mM. The total acetate concentration may be determined using methods known in the art, for example HPLC.

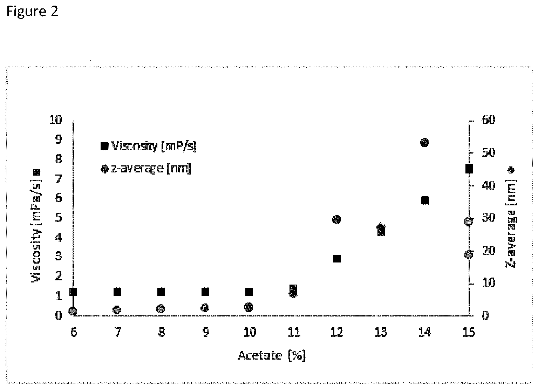

In the examples below, the viscosity of the liquid formulations of the present invention is shown to be dependent on the total acetate concentration. Preferably, the formulations have a viscosity between 0.8 and 2.0 mPa/sec as measured at 25°C. Conveniently, the viscosity may be measured by using *micro*VISC™. In parallel, the hydrodynamic radius may be measured using a Dynamic Light Scattering, DLS, Platereader (Wyatt DynaPro II). Samples were prepared having a drug substance (DS) of the GLP2-analogue containing 6% acetate and to mimic DS having 7.8-15% acetate, then acetate was added. Data from manufacturing formulations having varied the acetate concentration from 6.7-15% is shown below in Figure 2.

The liquid formulations according to the present invention are preferably an isosmotic liquid formulation. "Isosmotic" means that the formulations of the present invention have the same or a similar osmotic pressure with bodily fluids. Preferably, the formulations of the present invention have an osmolality of about 300 ± 60 mOsm as measured by an osmometer.

Additionally or alternatively, the present invention demonstrates that the formation of covalently linked oligomers of the GLP-2 analogue is inversely dependent on the concentration of the GLP-2 analogue in the formulation. As shown in the examples, this amount of covalently bonded oligomers can be determined using size exclusion chromatography and determining the area under the peaks for monomeric GLP-2 analogue and oligomers respectively. This can be done using a Dionex Ultimate3000 HPLC system, giving a linear gradient, at a flow rate of 0.5 mL/min was used for the analysis. The mobile phase consisted of 0.1% TFA in 45% acetonitrile and 55% Milli-Q water. A wavelength of 215 nm was used for detection. This means that the formulations of the present invention generally contain the GLP-2 analogue at a concentration of about 2 mg/mL to about 30 mg/mL, more preferably at a concentration of about 15 mg/mL to about 25 mg/mL, and most preferably at a concentration of about 20 mg/mL. In some aspects of the present invention, it is preferred that the concentration of the GLP-2 analogue is selected so that the formulation contains 10% or less, more preferably 5% or less, and more preferably 3% or less of the GLP-2 analogue in the form of covalently bonded oligomeric products, preferably after 18 months storage.

In some cases, the formulation of the present invention may be used in a once or twice daily dosage regime. In some cases, the formulation of the present invention may be used in a once or twice weekly dosage regime. Alternatively or additionally, the dosing regime of the GLP-2 analogues of the present invention may comprise a plurality or course of doses separated in time by 2 days, 2.5 days, 3 days, 3.5 days, 4 days, 5 days, 6 days, 7 days, 8 days, 9 days, 10 days, 11 days or 12 days. In a preferred embodiment, the doses are separated in time by 3 days, 3.5 days, 4 days, 5 days, 6 days, 7 days or 8 days. In a preferred embodiment, doses are separated in time by 3 days, 3.5 days, 4 days or 7 days. As will be appreciated in the art, the time between doses may be varied to some extent so that each and every doses is not separated by precisely the same time. This will often be directed under the discretion of the physician. Thus, doses may be separated in time by a clinically acceptable range of times, e.g. from about 2 days to about 10 days, or from about 3 or 4 days to about 7 or 8 days.

The formulations of the present invention are stable liquid pharmaceutical formulations of GLP-2 analogues. A "stable" formulation is one in which the peptide therein essentially retains its physical stability and/or chemical stability and/or biological activity upon storage. Preferably, the formulation essentially retains its physical and chemical stability, as well as its biological activity upon storage. The storage period is generally selected based on the intended shelf-life of the formulation. The formulations of the present invention are provided as stable liquid formulations, e.g. stable aqueous liquid formulations. Various analytical techniques for measuring protein stability are available in the art and are reviewed in Peptide and Protein Drug Delivery, 247-301, Vincent Lee Ed., Marcel Dekker, Inc., New York, N.Y., Pubs. (1991) and Jones, A. Adv. Drug Delivery Rev. 10: 29-90 (1993), for example. In the present invention, "stable" formulations include formulations in which at least 80%, more preferably at least 90%, more preferably at least 95%, more preferably at least 96%, more preferably at least 97%, more preferably at least 98%, and most preferably at least 99% of the GLP-2 analogue is active in the formulation after it has been stored at 2-8°C for at least 18 months.

Stability can be measured at a selected temperature for a selected time period, for example using elevated temperature to reduce the period over which a formulation is tested. Generally, storage at a temperature between 2 to 8°C denotes storage under normal refrigerated conditions. In certain embodiments, the formulation is stable under such conditions for at least 12 months, more preferably at least 18 months, more preferably at least 24 months. Stability can be evaluated qualitatively and/or quantitatively in a variety of different ways, including evaluation of aggregate formation (e.g. using size exclusion chromatography, by measuring turbidity, and/or by visual inspection); by assessing charge heterogeneity using cation exchange chromatography, image capillary isoelectric focusing (icIEF) or capillary zone electrophoresis; amino-terminal or carboxy-terminal sequence analysis; mass spectrometric analysis; SDS-PAGE analysis to compare reduced and intact antibody; peptide map (for example tryptic or LYS-C) analysis; evaluating biological activity or antigen binding function of the antibody; etc. Instability may involve any one or more of: aggregation, deamidation (e.g. Asn deamidation), oxidation (e.g. Met oxidation), isomerization (e.g. Asp isomeriation), clipping/hydrolysis/fragmentation (e.g. hinge region fragmentation), succinimide formation, unpaired cysteine(s), N-terminal extension, C-terminal processing, glycosylation differences, etc.

A peptide "retains its physical stability" in a pharmaceutical formulation if it shows no sign (or very little sign) of aggregation, precipitation and/or denaturation upon e.g. visual examination of colour and/or clarity, or as measured by UV light scattering, dynamic light scattering, circular dichroism, or by size exclusion chromatography and is considered to still retain its biological activity.

A peptide "retains its chemical stability" in a pharmaceutical formulation, if the chemical stability at a given time is such that the peptide is considered to still retain its biological activity as defined below. Chemical stability can be assessed by detecting and quantifying chemically altered forms of the peptide. Chemical alteration may involve isomerization, oxidation, size modification (e.g. clipping) which can be evaluated using HPLC or size exclusion chromatography, SDS-PAGE and/or mass spectrometry, for example. Other types of chemical alteration include charge alteration n (e.g. occurring as a result of deamidation) which can be evaluated by HPLC or ion-exchange chromatography or icIEF, for example.

### GLP-2 analogues

The GLP-2 analogues present in the formulations of the present invention have one or more amino acid substitutions, deletions, inversions, or additions compared with native GLP-2 and as defined above. This definition also includes the synonym terms GLP-2 mimetics and/or GLP-2 agonists. Further, the analogue of the present invention may additionally have chemical modification of one or more of its amino acid side groups, α-carbon atoms, terminal amino group, or terminal carboxylic acid group. A chemical modification includes, but is not limited to, adding chemical moieties, creating new bonds, and removing chemical moieties. Modifications at amino acid side groups include, without limitation, acylation of lysine ε-amino groups, N-alkylation of arginine, histidine, or lysine, alkylation of glutamic or aspartic carboxylic acid groups, and deamidation of glutamine or asparagine. Modifications of the terminal amino include, without limitation, the des-amino, N-lower alkyl, N-di-lower alkyl, and N-acyl modifications. Modifications of the terminal carboxy group include, without limitation, the amide, lower alkyl amide, dialkyl amide, and lower alkyl ester modifications. Preferably herein lower alkyl is C₁-C₄ alkyl. Furthermore, one or more side groups, or terminal groups, may be protected by protective groups known to the ordinarily-skilled peptide chemist. The α-carbon of an amino acid may be mono- or di-methylated.

In some aspects, the liquid formulations of the present invention employ a glucagon-like peptide 2 (GLP-2) analogue represented by the formula: 
R1-Z1-His-Gly-Glu-Gly-X5-Phe-Ser-Ser-Glu-Leu-X11-Thr-Ile-Leu-Asp-Ala-Leu-Ala-
Ala-Arg-Asp-Phe-Ile-Ala-Trp-Leu-Ile-Ala-Thr-Lys-Ile-Thr-Asp-Z2-R2
 wherein:
R¹ is hydrogen, C₁₋₄ alkyl (e.g. methyl), acetyl, formyl, benzoyl or trifluoroacetyl;
X5 is Ser or Thr;
X11 is Ala or Ser;
R² is NH₂ or OH; and
Z¹ and Z² are independently absent or a peptide sequence of 1-6 amino acid units of Lys;
or a pharmaceutically acceptable salt or derivative thereof.

In some embodiments of the present invention, in the above formula, X5 is Thr and/or X11 is Ala. Examples of these glucagon-like peptide 2 (GLP-2) analogues include:
ZP1848 H-HGEGTFSSELATILDALAARDFIAWLIATKITDKKKKKK-NH₂ (SEQ ID NO: 1)
ZP2949 H-HGEGTFSSELATILDALAARDFIAWLIATKITDKKK-OH (SEQ ID NO: 2);
ZP2711 H-HGEGTFSSELATILDALAARDFIAWLIATKITDKK-OH (SEQ ID NO: 3);
ZP2469 H-HGEGTFSSELATILDALAARDFIAWLIATKITDK-OH (SEQ ID NO: 4);
ZP1857 H-HGEGTFSSELATILDALAARDFIAWLIATKITD-NH₂ (SEQ ID NO: 5); or
ZP2530 H-HGEGTFSSELATILDALAARDFIAWLIATKITD-OH (SEQ ID NO: 6).

In an embodiment of the present invention, the glucagon-like peptide 2 (GLP-2) analogue is ZP1848 H-HGEGTFSSELATILDALAARDFIAWLIATKITDKKKKKK-NH₂ (SEQ ID NO: 1).

In some embodiments of the present invention, in the above formula X5 is Ser and/or X11 is Ser. Examples of these glucagon-like peptide 2 (GLP-2) analogues include:
ZP1846 H-HGEGSFSSELSTILDALAARDFIAWLIATKITDKKKKKK-NH₂(SEQ ID NO: 7);
ZP1855 H-HGEGSFSSELSTILDALAARDFIAWLIATKITD-NH₂ (SEQ ID NO: 8); or
ZP2242 H-HGEGSFSSELSTILDALAARDFIAWLIATKITDK-OH (SEQ ID NO: 9).

In an embodiment of the present invention, the glucagon-like peptide 2 (GLP-2) analogue is ZP1846 H-HGEGSFSSELSTILDALAARDFIAWLIATKITDKKKKKK-NH₂(SEQ ID NO: 7).

It should be understood that the peptides (drug substance) of the invention might also be provided in the form of a salt or other derivative. Salts include pharmaceutically acceptable salts, such as acid addition salts and basic salts. Examples of acid addition salts include hydrochloride salts, citrate salts, chloride salts and acetate salts. Preferably, the salt is acetate. Examples of basic salts include salts where the cation is selected from alkali metals, such as sodium and potassium, alkaline earth metals, such as calcium, and ammonium ions ⁺N (R³)₃(R⁴), where R³ and R⁴ independently designates optionally substituted C₁₋₆-alkyl, optionally substituted C₂₋₆-alkenyl, optionally substituted aryl, or optionally substituted heteroaryl. Other examples of pharmaceutically acceptable salts are described in "Remington's Pharmaceutical Sciences", 17th edition. Ed. Alfonso R. Gennaro (Ed.), Mark Publishing Company, Easton, PA, U.S.A., 1985 and more recent editions, and in the Encyclopaedia of Pharmaceutical Technology.

In preferred embodiments, the GLP-2 analogue of the invention is selected from the group consisting of ZP1848-acetate, ZP2949-acetate, ZP2711-acetate, ZP2469-acetate, ZP1857-acetate, ZP2530-acetate, ZP1846-acetate, ZP1855-acetate and ZP2242-acetate. In the present context, the term "ZP1848-acetate" refers to the ZP1848 molecule is in the form of an acetate salt.

In a preferred embodiment, the GLP-2 analogue is ZP1848-acetate or H-HGEGTFSSELATILDALAARDFIAWLIATKITDKKKKKK-NH₂ acetate (SEQ ID NO: 1).

Other derivatives of the GLP-2 analogues of the invention include coordination complexes with metal ions such as Mn²⁺ and Zn²⁺, esters such as *in vivo* hydrolysable esters, free acids or bases, hydrates, prodrugs or lipids. Esters can be formed between hydroxyl or carboxylic acid groups present in the compound and an appropriate carboxylic acid or alcohol reaction partner, using techniques well known in the art. Derivatives which as prodrugs of the compounds are convertible *in vivo* or *in vitro* into one of the parent compounds. Typically, at least one of the biological activities of compound will be reduced in the prodrug form of the compound, and can be activated by conversion of the prodrug to release the compound or a metabolite of it. Examples of prodrugs include the use of protecting groups which may be removed *in situ* releasing active compound or serve to inhibit clearance of the drug *in vivo.*

Z¹ and Z² are independently present and/or absent or a peptide sequence of 1-6 amino acid units of Lys, i.e. 1, 2, 3, 4, 5 or 6 Lys residues. The Lys residues may have either D- or L-configuration, but have an L-configuration. Particularly preferred sequences Z are sequences of four, five or six consecutive lysine residues, and particularly six consecutive lysine residues. Exemplary sequences Z are shown in WO 01/04156. In certain embodiments, Z¹ is absent. In such cases, Z² may be either present or absent.

### Formulations of the GLP-2 analogues

The formulation of the GLP-2 analogues is a ready-to-use formulation. The term "ready-to-use" as used herein refers to a formulation that does not require constitution or dilution with a prescribed amount of diluent, e.g., water for injection or other suitable diluent, before use by the designated route of administration.

As described herein, the liquid formulations of the GLP-2 analogues of the present invention include a buffer, a non-ionic tonicity modifier and arginine q.s. to provide the pH of the final formulation. In accordance with normal pharmaceutical practice, the formulations of the present invention are sterile and/or free from reducing agent. In some cases, the liquid formulations of the present invention are aqueous, liquid formulations. In some cases, the liquid formulations of the present invention are non-aqueous, liquid formulations.

The term "buffer" as used herein denotes a pharmaceutically acceptable excipient which stabilizes the pH of a pharmaceutical formulation. Suitable buffers are well known in the art and can be found in the literature. The screening experiments in the examples show that the formulations of the present invention preferably include a buffer selected from histidine buffer, acetate buffer or mesylate buffer as these buffers provided stable formulations in which the GLP-2 analogues dissolved and did not become viscous, cloudy or precipitate the peptide drug. In preferred embodiments, the buffer is a histidine buffer, e.g. L-histidine. Generally, the buffer will be present at a concentration of about 5 mM to about 50 mM, more preferably at a concentration of about 5 mM to about 25 mM, and most preferably at a concentration of about 15 mM. Based on the experiments in the present application, preferably the buffer is not a phosphate buffer, a citrate buffer, citrate/Tris buffer and/or succinate buffer.

The term "tonicity modifier" as used herein denotes pharmaceutically acceptable tonicity agents that are used to modulate the tonicity of the formulation. The formulations of the present invention are preferably isosmotic, that is they have an osmotic pressure that is substantially the same as human blood serum. The tonicity modifiers used in the formulations are preferably non-ionic tonicity modifiers and are preferably selected from the group consisting of mannitol, sucrose, glycerol and sorbitol. A preferred non-ionic tonicity modified is mannitol, e.g. D-mannitol. The concentration of the tonicity modifier will be dependent on the concentration of other components of the formulation, especially where the formulation is intended to be isosmotic. Typically, the non-ionic tonicity modifier will be employed at a concentration of about 90 mM to about 360 mM, more preferably at a concentration of about 150 mM to about 250 mM, and most preferably at a concentration of about 230 mM.

Generally, the components and amounts of the liquid formulations of the present invention are chosen to provide a formulation with a pH of about 6.6 to about 7.4, more preferably a pH of about 6.8 to about 7.2, and most preferably a pH of about 7.0. Arginine may be added *quantum sufficit* (*q.s.*) to adjust pH so that it is within a desired pH range.

In one embodiment, the liquid formulations of the present invention consists of the GLP-2 analogue at a concentration of about 2 mg/mL to about 30 mg/mL, a buffer selected from the group consisting of a histidine buffer, mesylate buffer and acetate buffer, the buffer being present at a concentration of about 5 mM to about 50 mM, a non-ionic tonicity modifier selected from the group consisting of mannitol, sucrose, glycerol and sorbitol at a concentration of about 90 mM to about 360 mM, arginine q.s. to provide a pH of about 6.6 to about 7.4.

In a further embodiment, the liquid formulations of the present invention comprises the GLP-2 analogue at a concentration of about 20 mg/mL, histidine buffer at a concentration of about 15 mM, mannitol at a concentration of about 230 mM, and arginine q.s. to provide a pH of about 7.0.

In a further embodiment, the liquid formulations of the present invention comprises the GLP-2 analogue at a concentration of about 20 mg/mL, histidine buffer at a concentration of about 15 mM, mannitol at a concentration of about 230 mM and the pH is about 7.0.

In a further embodiment, the liquid formulations of the present invention comprises ZP1848-acetate or H-HGEGTFSSELATILDALAARDFIAWLIATKITDKKKKKK-NH₂ acetate (SEQ ID NO: 1) at a concentration of about 20 mg/mL, histidine buffer at a concentration of about 15 mM, mannitol at a concentration of about 230 mM, and arginine q.s. to provide a pH of about 7.0.

In a further embodiment, the liquid formulations of the present invention comprises ZP1848-acetate or H-HGEGTFSSELATILDALAARDFIAWLIATKITDKKKKKK-NH2 acetate (SEQ ID NO: 1) at a concentration of about 20 mg/mL, histidine buffer at a concentration of about 15 mM, mannitol at a concentration of about 230 mM and the pH is about 7.0.

In a further embodiment, the liquid formulations of the present invention comprises ZP1846 H-HGEGSFSSELSTILDALAARDFIAWLIATKITDKKKKKK-NH2 (SEQ ID NO: 7); at a concentration of about 20 mg/mL, histidine buffer at a concentration of about 15 mM, mannitol at a concentration of about 230 mM, and arginine q.s. to provide a pH of about 7.0.

In a further embodiment, the liquid formulations of the present invention comprises ZP1846 H-HGEGSFSSELSTILDALAARDFIAWLIATKITDKKKKKK-NH₂(SEQ ID NO: 7); at a concentration of about 20 mg/mL, histidine buffer at a concentration of about 15 mM, mannitol at a concentration of about 230 mM and the pH is about 7.0.

In a further embodiment, the liquid formulation is selected from the group consisting of an aqueous liquid formulation, a liquid formulation in various hydrophilic or hydrophobic solvents, an emulsion and a liquid suspension. In a preferred embodiment, the liquid formulation is an aqueous liquid formulation.

Preferably, the glucagon-like peptide 2 (GLP-2) analogue are administered to patients parenterally, preferably by injection, most typically by subcutaneous injection, intramuscular injection, intravenous injection or intraperitoneal injection. Administration by subcutaneous injection is preferred. The injection may be carried out by a physician, nurse or other healthcare professional, or may be self-administered by the patient. As set out herein, in some aspects, the formulations of the present invention have a viscosity that facilitates loading of the formulation into a pre-filled syringe, an injection pen or other injector device. This may have the advantage of pre-determining the dose of the formulation administered to the patient, e.g. without the need for measurement from a multi-use vial. Accordingly, in other aspects, the present invention provides an article of manufacture or a kit comprising a container holding the stable, such as e.g. an aqueous stable pharmaceutical formulation of the GLP-2 analogue according to the present invention or a pre-filled syringe or injector device or injector pen containing an aqueous liquid formulation comprising the GLP-2 analogue according to the present invention.

### Medical Conditions

The GLP-2 analogue formulations of the present invention are useful as a pharmaceutical agent for preventing or treating an individual suffering from gastro-intestinal disorders, including the upper gastrointestinal tract of the oesophagus by administering an effective amount of a GLP-2 analogue, or a salt thereof as described herein. The stomach and intestinal-related disorders include ulcers of any aetiology (e.g., peptic ulcers, drug-induced ulcers, ulcers related to infections or other pathogens), digestion disorders, malabsorption syndromes, short-bowel syndrome, cul-de-sac syndrome, inflammatory bowel disease, celiac sprue (for example arising from gluten induced enteropathy or celiac disease), tropical sprue, hypogammaglobulinemic sprue, enteritis, ulcerative colitis, small intestine damage, and chemotherapy induced diarrhoea/mucositis (CID).

As mentioned above, in general individuals who would benefit from increased small intestinal mass and consequent and/or maintenance of normal small intestine mucosal structure and function are candidates for treatment with the present GLP-2 analogues. Particular conditions that may be treated with GLP-2 analogue include the various forms of sprue including celiac sprue which results from a toxic reaction to alpha-gliadin from heat and may be a result of gluten-induced enteropathy or celiac disease, and is marked by a significant loss of villae of the small bowel; tropical sprue which results from infection and is marked by partial flattening of the villae; hypogammaglobulinemic sprue which is observed commonly in patients with common variable immunodeficiency or hypogammaglobulinemia and is marked by significant decrease in villus height. The therapeutic efficacy of the GLP-2 analogue treatment may be monitored by enteric biopsy to examine the villus morphology, by biochemical assessment of nutrient absorption, by patient weight gain, or by amelioration of the symptoms associated with these conditions.

Another particular condition which may be treated with the GLP-2 analogues of the invention, or for which the GLP-2 analogues may be useful therapeutically and/or prophylactically is short bowl syndrome (SBS), also known as short gut syndrome or simply short gut, which results from surgical resection, congenital defect or disease-associated loss of absorption in the bowel in which patients are subsequently unable to maintain fluid, electrolyte, and nutrient balances on a conventional diet. Despite an adaptation that occurs generally in the two years after resection, SBS patients have reduced dietary uptake and fluid loss.

Other conditions that may be treated with the GLP-2 analogues of the invention, or for which the GLP-2 analogues may be useful prophylactically, include in addition to the above mentioned radiation enteritis, infectious or post-infectious enteritis, and small intestinal damage due to cancer-chemotherapeutic or toxic agents.

The GLP-2 analogues may also be used for the treatment of malnutrition, for example cachexia and anorexia.

A particular embodiment of the invention is concerned with using the present peptides for the prevention and/or treatment of intestinal damage and dysfunction. Such damage and dysfunction is a well-known side effect of cancer-chemotherapy treatment. Chemotherapy administration is frequently associated with unwanted side effects related to the gastronintestinal system such as mucositis, diarrhoea, bacterial translocation, malabsorption, abdominal cramping, gastrointestinal bleeding and vomiting. These side effects are clinical consequences of the structural and functional damage of the intestinal epithelium and frequently make it necessary to decrease the dose and frequency of chemotherapy.

Administration of the present GLP-2 peptide analogues may enhance trophic effect in the intestinal crypts and rapidly provide new cells to replace the damaged intestinal epithelium following chemotherapy. The ultimate goal achieved by administering the present peptides is to reduce the morbidity related to gastrointestinal damage of patients undergoing chemotherapy treatment while creating the most optimal chemotherapy regime for the treatment of cancer. Concomitant prophylactic or therapeutic treatment may be provided in accordance with the present invention to patients undergoing or about to undergo radiation therapy.

The stem cells of the small intestinal mucosa are particularly susceptible to the cytotoxic effects of chemotherapy due to their rapid rate of proliferation (Keefe et al., Gut, 47: 632-7, 2000). Chemotherapy-induced damage to the small intestinal mucosa is clinically often referred to as gastrointestinal mucositis and is characterized by absorptive and barrier impairments of the small intestine. For example, it has been shown that, the broadly used chemotherapeutic agents, 5-FU, irinotecan and methothrexate increase apoptosis leading to villus atrophy and crypt hypoplasia in the small intestine of rodents (Keefe et al., Gut 47: 632-7, 2000; Gibson et al., J Gastroenterol. Hepatol. Sep;18(9):1095-1100, 2003; Tamaki et al., J. Int. Med. Res. 31(1):6-16, 2003). Chemotherapeutic agents have been shown to increase apoptosis in intestinal crypts at 24 hours after administration and subsequently to decrease villus area, crypt length, mitotic count per crypt, and enterocyte height three days after chemotherapy in humans (Keefe et al., Gut, 47: 632-7, 2000). Thus, structural changes within the small intestine directly lead to intestinal dysfunction and in some cases diarrhoea.

Gastrointestinal mucositis after cancer chemotherapy is an increasing problem that is essentially untreatable once established, although it gradually remits. Studies conducted with the commonly used cytostatic cancer drugs 5-FU and irinotecan have demonstrated that effective chemotherapy with these drugs predominantly affects structural integrity and function of the small intestine while the colon is less sensitive and mainly responds with increased mucus formation (Gibson et al., J. Gastroenterol. Hepatol. Sep;18(9):1095-1100, 2003; Tamaki et al., J Int. Med. Res. 31(1):6-16, 2003).

The formulations of the present invention comprising GLP-2 analogues may be useful in the prevention and/or treatment of gastrointestinal injury and side effects of chemotherapeutic agents. This potentially important therapeutic application may apply to currently used chemotherapeutic agents such as but not limited to: 5-FU, Altretamine, Bleomycin, Busulfan, Capecitabine, Carboplatin, Carmustine, Chlorambucil, Cisplatin, Cladribine, Crisantaspase, Cyclophosphamide, Cytarabine, Dacarbazine, Dactinomycin, Daunorubicin, Docetaxel, Doxorubicin, Epirubicin, Etoposide, Fludarabine, Fluorouracil, Gemcitabine, Hydroxycarbamide, Idarubicin, Ifosfamide, Irinotecan, Liposomal doxorubicin, Leucovorin, Lomustine, Melphalan, Mercaptopurine, Mesna, Methotrexate, Mitomycin, Mitoxantrone, Oxaliplatin, Paclitaxel, Pemetrexed, Pentostatin, Procarbazine, Raltitrexed, Streptozocin, Tegafur-uracil, Temozolomide, Thiotepa, Tioguanine/Thioguanine, Topotecan, Treosulfan, Vinblastine, Vincristine, Vindesine, Vinorelbine, Bleomycin, Busulfan, Capecitabine, Carboplatin, Carmustine, Chlorambucil, Cisplatin, Cladribine, Crisantaspase, Cyclophosphamide, Cytarabine, Dacarbazine, Dactinomycin, Daunorubicin, Docetaxel, Doxorubicin, Epirubicin, Etoposide, Fludarabine, Fluorouracil, Gemcitabine, Hydroxycarbamide, Idarubicin, Ifosfamide, Irinotecan, Liposomal doxorubicin, Leucovorin, Lomustine, Melphalan, Mercaptopurine, Methotrexate, Mitomycin, Mitoxantrone, Oxaliplatin, Paclitaxel, Pemetrexed, Pentostatin, Procarbazine, Raltitrexed, Streptozocin, Tegafur-uracil, Temozolomide, Thiotepa, Tioguanine/Thioguanine, Topotecan, Treosulfan, Vinblastine, Vincristine, Vindesine, and Vinorelbine.

### Delivery of the Formulations

In some aspects, the present invention relates to a ready-to-use formulation of GLP-2 analogues, intended for parenteral administration, and suitable for use in e.g. vials, pre-filled syringes, infusion pumps, wearable injectors, disposable auto-injectors or adjustable dose auto-injectors.

### Examples

The following examples are provided to illustrate preferred aspects of the invention and are not intended to limit the scope of the invention. The GLP-2 analogues administered according to the dosage regimes described herein can be made according to the methods such as solid phase peptide synthesis described in WO 2006/117565, the content of which is expressly incorporated by reference in its entirety.

### Example 1. Synthesis of ZP1848 and similar GLP-2 analogues

ZP1848 peptide was synthesized using a Solid Phase Peptide Synthesis (SPPS) approach and standard Fmoc coupling methodologies. After completed synthesis, the peptide sequence was deprotected and cleaved from the solid support, and the crude peptide was purified using preparative reverse phase HPLC. The peptide was converted to the acetate salt form or other acceptable salts and lyophilised to provide the final ZP1848 drug substance.

This synthesis and purification protocol may be adapted for making other GLP-2 analogues used in the formulations of the present invention.

### Example 2. Investigating the formation of covalently bound oligomers in pharmaceutical formulations of GLP-2 analogue ZP1848

### Materials and Methods

A Dionex Ultimate3000 HPLC system, giving a linear gradient, at a flow rate of 0.5 mL/min was used for the analysis. The mobile phase consisted of 0.1% TFA in 45% acetonitrile and 55% Milli-Q water. A wavelength of 215 nm was used for detection. An injection amount was 4 µg of peptide. The column used for the separation of the covalently formed peptides was a TSKgel SuperSW2000 (TSK BioScience) with a 4 µm particle size and dimensions of 300* 4.6 mm. The overall runtime was 25 minutes.

### Results and Discussion

It is known in the art that increasing the concentration of peptide or protein drugs in a liquid formulation increases the concentration of dimer, trimers and higher order oligomers as a result of mass action effects leading to a higher probability of covalent reactions (see van Maarschalkerweerd et al., Intrinsically Disord. Proteins. 2015; 3(1): e1071302). Thus, the formation of covalent high molecular weight degradation products (cHMWDP) increases as a function of drug substance concentration and has the effect of reducing the amount of biologically active monomeric peptide available in the formulation. This was therefore investigated in formulations of the GLP-2 analogue, ZP1848.

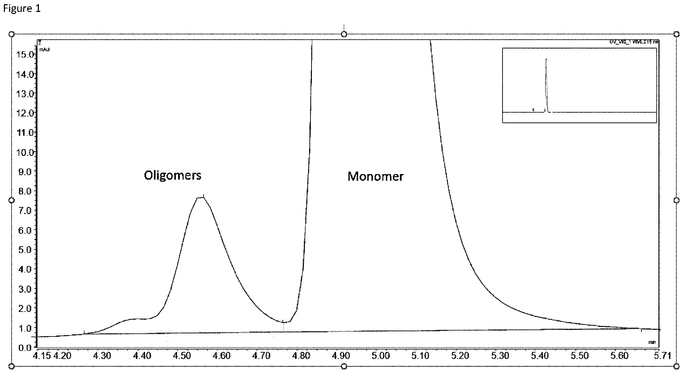

A typical chromatogram on the separation of the oligomers from the ZP1848 monomer is shown in Figure 1. The oligomers of ZP1848 are well separated from the ZP1848 monomer and are all integrated as one peak. The area percentage of the peaks was used to quantify the amount of oligomers, in particular covalently linked dimers and trimers.

Formulations containing 0.2, 2 and 20 mg/mL ZP1848 in the same formulation was analysed after 24 months of storage. It is primarily formation of dimers (two covalently linked ZP1848 molecules), but also to some extent trimers (verified by LC-MS). The formulation containing 0.2 mg/mL has 2.6% oligomers, 2 mg/mL has 1.91 % and 20.0 mg/mL has 1.35%. The initial value of the amount of oligomer was less than 0.1%.

**Table 1**

| **Drug product concentration, ZP1848** | **Covalently linked oligomers** |
|---|---|
| 0.2 mg/mL | 2.60% |
| 2 mg/mL | 1.91% |
| 20 mg/mL | 1.35% |

During long term storage at 2-8°C of ZP1848 (glepaglutide), it has surprisingly been found that the formation of covalently bound oligomers is concentration dependent, but contrary to the general expectation, the concentration dependence for oligomer formation is inversely dependent on increasing concentration of the GLP-2 analogue. Without wishing to be bound by any particular theory, the present inventors believe that the reduction in the formation of covalently linked oligomers as drug concentration increases is a result of the lysine tail of the GLP-2 analogue promoting a competing reaction leading to the formation of higher order species in which the GLP-2 analogue molecules are weakly associated together, rather than being covalently linked. This means that these weakly associated species are capable of dissociating to release biologically active monomer, rather than causing a loss of active species, as happens when the covalently bound oligomers form.

### Example 3: Buffer screening for formulations of GLP-2 analogue ZP1848

### Materials and Methods

The buffer solutions listed in the Table 2 below were prepared. pH of the buffers were adjusted with 1 M HCl/ 1 M NaOH. ZP1848 peptide was dissolved in the relevant buffer at 80% of the final sample volume to give 4 mg/mL in the final formulation. If necessary, pH was then adjusted to the desired formulation pH using either 200 mM acetic acid or 100 mM L-arginine. Buffer solution was added up to the final volume. Each formulation was filled in appropriate vials (1 mL/vial) for stability testing. Vials were visually inspected for clarity and viscosity.

### Results and Discussion

Visual appearance showed that all formulations containing citrate buffer, citrate/Tris buffer or succinate buffer were viscous and/or turbid (see Table). Acetate buffer (20 mM, pH 5), mesylate buffer (20 mM, pH 6) histidine buffer (15 mM, pH 7) and histidine-arginine (15+5 mM, pH 7) produced formulations which passed visual inspection as being clear and non-viscous.

**Table 2**

| **Formulation** | **pH** | **Buffer** | **Buffer concentration (mM)** | **Visual inspection Clear and non-viscous?** |
|---|---|---|---|---|
| 1 | 4.0 | Citrate-TRIS | 20 | No |
| 2 | 5.0 | Citrate-TRIS | 20 | No |
| 3 | 6.0 | Citrate-TRIS | 20 | No |
| 4 | 7.0 | Citrate-TRIS | 20 | No |
| 5 | 8.0 | Citrate-TRIS | 20 | No |
| 6 | 5.0 | Succinate | 20 | No |
| 7 | 5.0 | Acetate | 20 | Yes |
| 8 | 5.0 | Histidine | 20 | Yes |
| 9 | 6.0 | Succinate | 20 | No |
| 10 | 6.0 | Mesylate | 20 | Yes |
| 11 | 6.0 | Histidine | 20 | Yes |
| 12 | 7.0 | Citrate | 20 | No |
| 13 | 7.0 | TRIS | 20 | Yes |
| 14 | 7.0 | Histidine + Arginine | 15 + 5 | Yes |

### Example 4: Phosphate buffer incompatibility with GLP-2 analogue ZP1848

### Material and Methods

Stock solutions of mannitol (700 mM), phosphate buffer (200 mM) and ZP1848 peptide (60.2 mg/ml) in water (Milli-Q) were prepared. Stock solutions were mixed in amounts appropriate to give the formulations shown in the Table 3 below. Water was added up to 90% of final volume. If necessary, pH was then adjusted to the desired formulation pH using 1 M acetic acid/0.5 M L-arginine. Water was added up to the final volume (4.9mL). Sample containers were visually inspected for clarity and viscosity after 24 hours at room temperature.

### Results and Discussion

Visual inspection showed that formulations of ZP1848 at 20 mg/mL at pH 6.5-7.5 containing 20-50 mM phosphate buffer were turbid and/or highly viscous after 24 hrs at room temperature.

**Table 3**

| **ZP1848 concentration [mg/mL]** | **pH** | **Phosphate [mM]** | **Mannitol [mM]** | **Visual inspection Clear and non-viscous?** |
|---|---|---|---|---|
| 0 | 7.0 | 20 | 230 | Yes |
| 20 mg/mL | 6.5 | 20 | 230 | No |
| 20 mg/mL | 7.0 | 20 | 230 | No |
| 20 mg/mL | 7.0 | 50 | 230 | No |
| 20 mg/mL | 7.5 | 20 | 230 | No |

### Example 5: Effect of acetate content on viscosity of formulations of GLP-2 analogue ZP1848

A study was carried out to determine the effect of the acetate content on the viscosity of the ZP1848 formulation.

### Material and Methods

Samples were prepared using a drug substance (DS) of the GLP-2 analogue (ZP1848) containing 6% acetate. Acetate was added to explore the effects of increased acetate content in the range 7.8-15% acetate (see Table 4).

### Stock solutions of mannitol (700 mM), acetic acid (1000 mM), histidine (200 mM) and

ZP1848 peptide (60 mg/ml) in water (Milli-Q) were prepared. Stock solutions were mixed in amounts appropriate to give the formulations shown in the Table 4 below. Water was added up to 90% of final volume. If necessary, pH was then adjusted to the desired formulation pH using 250 mM arginine. Water was added up to the final volume. Each formulation was filled in appropriate vials for stability testing.

Vials were visually inspected for clarity and viscosity. The viscosity was measured using a *micro*VISC™ viscosimeter. The hydrodynamic radius was measured using a Wyatt DynaPro II Dynamic Light Scattering (DLS) Platereader. The sample size loaded on the plates was 170 µl.

**Table 4: Preparations containing 20 mg/mL of ZP1848 at pH 7 with different acetate concentrations**

| **Formulation #** | **Mannitol [mM]** | **Histidine [mM]** | **Acetate %** | **Acetate [mM]** |
|---|---|---|---|---|
| 1 | 230 | 15 | 6 | 20.3 |
| 2 | 230 | 15 | 7 | 23.7 |
| 3 | 230 | 15 | 8 | 27.1 |
| 4 | 230 | 15 | 9 | 30.5 |
| 5 | 230 | 15 | 10 | 33.9 |
| 6 | 230 | 15 | 11 | 37.3 |
| 7 | 230 | 15 | 12 | 40.7 |
| 8 | 230 | 15 | 13 | 44.0 |
| 9 | 230 | 15 | 14 | 47.4 |
| 10 | 230 | 15 | 15 | 50.8 |

The viscosity and hydrodynamic radius of the formulations with varying acetate concentration are shown in Figure 2. The results demonstrate that the viscosity of the ZP1848 formulation unexpectedly increases in a non-linear manner at higher acetate concentration. It is therefore advantageous for controlling the viscosity at a low/unchanged level if the total acetate concentration in the formulation is less than or equal to 11% acetate per mg GLP-2 analogue, as this opens up the possibility of providing the formulations of the GLP-2 analogue in the form of a drug delivery device.

While the present invention has been described in conjunction with the embodiments described above, many equivalent modifications and variations will be apparent to those skilled in the art when given this disclosure. Accordingly, the embodiments of the invention set forth are considered to be illustrative and not limiting. Various changes to the described embodiments may be made without departing from the spirit and scope of the invention. All documents cited herein are expressly incorporated by reference in their entirety for all purposes.

## Claims

1. A stable liquid pharmaceutical formulation, the formulation comprising a glucagon-like peptide 2 (GLP-2) analogue, wherein the GLP-2 analogue is represented by the formula: 
R1-Z1-His-Gly-Glu-Gly-X5-Phe-Ser-Ser-Glu-Leu-X11-Thr-Ile-Leu-Asp-Ala-Leu-Ala-
Ala-Arg-Asp-Phe-Ile-Ala-Trp-Leu-Ile-Ala-Thr-Lys-Ile-Thr-Asp-Z2-R2
 wherein:
R¹ is hydrogen, C₁₋₄ alkyl (e.g. methyl), acetyl, formyl, benzoyl or trifluoroacetyl;
X5 is Ser or Thr;
X11 is Ala or Ser;
R² is NH₂ or OH; and
Z¹ and Z² are independently absent or a peptide sequence of 1-6 amino acid units of Lys;
or a pharmaceutically acceptable salt or derivative thereof;
wherein the formulation comprises:
(a) the GLP-2 analogue at a concentration of about 2 mg/mL to about 30 mg/mL;
(b) a buffer selected from the group consisting of a histidine buffer, mesylate buffer and acetate buffer, the buffer being present at a concentration of about 5 mM to about 50 mM;
(c) a non-ionic tonicity modifier selected from the group consisting of mannitol, sucrose, glycerol and sorbitol at a concentration of about 90 mM to about 360 mM; and
(d) arginine q.s. to provide a formulation having a pH of about 6.6 to about 7.4;
wherein the formulation contains 5% or less of the GLP-2 analogue in the form of covalently bonded oligomeric products.

2. The formulation according to claim 1, wherein the total acetate concentration arising from the GLP2 analogue in the formulation is less than or equal to 11% acetate per mg GLP-2 analogue.

3. The formulation according to claim 1 or claim 2, wherein formation of covalently linked oligomers of the GLP-2 analogue is inversely dependent on the concentration of the GLP-2 analogue in the formulation.

4. The formulation according to any one of claims 1 to 3, wherein the formulation is an aqueous formulation.

5. The formulation according to any one of claims 1 to 4, wherein the formulation is stable for at least 18 months when stored at 2-8°C.

6. The formulation according to any one of the preceding claims, wherein the formulation has a viscosity between 0.8 and 2.0 mPa/sec measured at 25°C.

7. The formulation according to any one of the preceding claims, wherein the GLP-2 analogue is present in the formulation at a concentration of about 15 mg/mL to about 25 mg/ml.

8. The formulation according to any one of the preceding claims, wherein the formulation is a ready-to-use formulation.

9. The formulation according to any one of the preceding claims, wherein the GLP-2 analogue is present in the formulation at a concentration of about 20 mg/mL.

10. The formulation according to any one of the preceding claims, wherein the buffer is present in the formulation at a concentration of about 5 mM to about 25 mM.

11. The formulation according to any one of the preceding claims, wherein the buffer is a histidine buffer.

12. The formulation according to claim 11, wherein the histidine buffer is present in the formulation at a concentration of about 15 mM.

13. The formulation according to any one of the preceding claims, wherein the non-ionic tonicity modifier is present in the formulation at a concentration of about 150 mM to about 250 mM.

14. The formulation according to any one of the preceding claims, wherein the non-ionic tonicity modifier is mannitol.

15. The formulation according to claim 14, wherein the mannitol is present in the formulation at a concentration of about 230 mM.

16. The formulation according to any one of the preceding claims, wherein the formulation has a pH of about 6.8 to about 7.2.

17. The formulation according to any one of the preceding claims, wherein the formulation has a pH of about 7.0.

18. The formulation according to any one of the preceding claims, wherein the formulation consists of the GLP-2 analogue at a concentration of about 2 mg/mL to about 30 mg/ml, a buffer selected from the group consisting of a histidine buffer, mesylate buffer and acetate buffer, the buffer being present at a concentration of about 5 mM to about 50 mM, a non-ionic tonicity modifier selected from the group consisting of mannitol, sucrose, glycerol and sorbitol at a concentration of about 90 mM to about 360 mM, arginine q.s. to provide a pH of about 6.6 to about 7.4.

19. The formulation according to any one of the preceding claims, wherein the formulation comprises the GLP-2 analogue at a concentration of about 20 mg/mL, histidine buffer at a concentration of about 15 mM, mannitol at a concentration of about 230 mM, and arginine q.s. to provide a pH of about 7.0.

20. The formulation according to any one of the preceding claims, wherein the histidine buffer is L-histidine.

21. The formulation according to any one of the preceding claims, wherein the mannitol is D-mannitol.

22. The formulation according to any one of the preceding claims, wherein the arginine acid is L-arginine/acetic acid.

23. The formulation according to any one of the preceding claims, wherein the formulation is free of reducing agent.

24. The formulation according to any one of the preceding claims, wherein the formulation is stable at 2-8°C for at least 6 months, at least 12 months, at least 18 months or at least 24 months.

25. The formulation according to claim 24, wherein the GLP-2 analogue in the formulation retains at least about 90% of its biological activity after 18 months of storage 2-8°C.

26. The formulation according to any one of the preceding claims which is sterile.

27. The formulation according to any one of the preceding claims, wherein the formulation is administration to a subject by injection.

28. The formulation according to claim 27, wherein the injection is a subcutaneous injection.

29. The formulation according to any one of the preceding claims, wherein the GLP-2 analogue is provided as an acetate salt.

30. The formulation according to any one of the preceding claims, wherein the GLP-2 analogue is ZP1848 or ZP1848-acetate.

31. The formulation according to any one of the preceding claims, wherein the formulation consist of ZP1848-acetate at a concentration of about 20 mg/mL, histidine buffer at a concentration of about 15 mM, mannitol at a concentration of about 230 mM, and arginine q.s. to provide a pH of about 7.0.

32. An article of manufacture or a kit comprising a container holding the stable pharmaceutical formulation of any one of claims 1 to 31.

33. A delivery device containing a liquid formulation comprising a GLP-2 analogue of any one of claims 1 to 31.

34. The delivery device of claim 33, wherein the delivery device is pre-filled syringe, an injector device, an injector pen, an adjustable dose auto-injector, a disposable auto-injector, a wearable injector, an infusion pump.

35. A formulation of the glucagon-like peptide 2 (GLP-2) analogue of any one of claims 1 to 31 for use in therapy.

36. A formulation of the glucagon-like peptide 2 (GLP-2) analogue of any one of claims 1 to 31 for use in a method for the treatment and/or prevention of a stomach and bowel-related disorder in a human patient.

37. The formulation of the glucagon-like peptide 2 (GLP-2) analogue for use in the method of treatment and/or prevention of claim 36, wherein the stomach and bowel-related disorder is ulcers, digestion disorders, malabsorption syndromes, short-gut syndrome, cul-de-sac syndrome, inflammatory bowel disease, celiac sprue (for example arising from gluten induced enteropathy or celiac disease), tropical sprue, hypogammaglobulinemic sprue, enteritis, regional enteritis (Crohn's disease), ulcerative colitis, small intestine damage or short bowel syndrome (SBS).

38. The formulation of the glucagon-like peptide 2 (GLP-2) analogue for use in the method of treatment and/or prevention of claim 37, wherein the stomach and bowel-related disorder is short bowel syndrome.

39. The formulation of the glucagon-like peptide 2 (GLP-2) analogue for use in a method for the treatment according to claim 36, wherein the stomach and bowel-related disorder is radiation enteritis, infectious or post-infectious enteritis, or small intestinal damage due to toxic or other chemotherapeutic agents.

40. The formulation of the glucagon-like peptide 2 (GLP-2) analogue for use in a method for the treatment according to claim 39, wherein treatment with the GLP-2 analogue is combined with one or more anti-cancer therapies.

41. The formulation of the glucagon-like peptide 2 (GLP-2) analogue for use in a method for the treatment according to claim 40, wherein treatment the anti-cancer therapy comprises administering one or more chemotherapeutic agent(s) to the patient or treating the patient with radiation therapy.

42. The formulation of the glucagon-like peptide 2 (GLP-2) analogue for use in a method for the treatment according to claim 40 or claim 41, wherein the formulation is used in the treatment and/or prevention of a side effect of chemotherapy or radiation treatment.

43. The formulation of the glucagon-like peptide 2 (GLP-2) analogue for use in a method for the treatment according to claim 42, wherein the side effect of chemotherapy is diarrhoea, abdominal cramping, vomiting or structural and functional damage of the intestinal epithelium resulting from chemotherapy treatment.

44. The formulation of the glucagon-like peptide 2 (GLP-2) analogue for use in a method for the treatment according to any one of claims 40 to 43, wherein the human patient is a patient having SBS-intestinal failure.

45. The formulation of the glucagon-like peptide 2 (GLP-2) analogue for use in a method for the treatment according to any one of claims 40 to 44, wherein the human patient is a patient being on the border between being a patient having SBS-intestinal insufficiency and SBS-intestinal failure.

46. The formulation of the glucagon-like peptide 2 (GLP-2) analogue for use in a method for the treatment according to any one of claims 35 to 45, wherein the method comprises administering the GLP-2 analogue to the patient once weekly.

47. The formulation of the glucagon-like peptide 2 (GLP-2) analogue for use in a method for the treatment according to any one of claims 29 to 43, wherein the method comprises administering the GLP-2 analogue to the patient twice weekly.

48. A process for producing a stable liquid pharmaceutical formulation comprising a glucagon-like peptide 2 (GLP-2) analogue, wherein the GLP-2 analogue is represented by the formula: 
R1-Z1-His-Gly-Glu-Gly-X5-Phe-Ser-Ser-Glu-Leu-X11-Thr-Ile-Leu-Asp-Ala-Leu-Ala-
Ala-Arg-Asp-Phe-Ile-Ala-Trp-Leu-Ile-Ala-Thr-Lys-Ile-Thr-Asp-Z2-R2
 wherein:
R¹ is hydrogen, C₁₋₄ alkyl (e.g. methyl), acetyl, formyl, benzoyl or trifluoroacetyl
X5 is Ser or Thr
X11 is Ala or Ser
R² is NH₂ or OH;
Z¹ and Z² are independently absent or a peptide sequence of 1-6 amino acid units of Lys; or a pharmaceutically acceptable salt or derivative thereof;
wherein the process comprising formulating (a) the GLP-2 analogue at a concentration of about 2 mg/mL to about 30 mg/mL, (b) with a buffer selected from the group consisting of a histidine buffer, mesylate buffer and acetate buffer, the buffer being present at a concentration of about 5 mM to about 50 mM; (c) with a non-ionic tonicity modifier selected from the group consisting of mannitol, sucrose, glycerol and sorbitol, the non-ionic tonicity modifier being present at a concentration of about 90 mM to about 360 mM; and (d) with arginine q.s. to provide a formulation having a pH of about 6.6 to about 7.4;
wherein the formulation contains 5% or less of the GLP-2 analogue in the form of covalently bonded oligomeric products.

49. The process according to claim 48, wherein formation of covalently linked oligomers of the GLP-2 analogue is inversely dependent on the concentration of the GLP-2 analogue in the formulation.

50. The process according to claim 48 or claim 49, wherein the formulation is stable for at least 18 months when stored at 2-8°C.

51. The process according to any one of claims 48 to 50, wherein the total acetate concentration arising from the GLP2 analogue in the formulation is less than or equal to 11% acetate per mg GLP-2 analogue.

52. The process according to any one of claims 48 to 51, wherein the formulation has a viscosity greater than 0.8 and lower than or equal to 2.0 mPa/sec measured at 25°C.

53. Use of a formulation comprising a glucagon-like peptide 2 (GLP-2) analogue, wherein the GLP-2 analogue is represented by the formula: 
R1-Z1-His-Gly-Glu-Gly-X5-Phe-Ser-Ser-Glu-Leu-X11-Thr-Ile-Leu-Asp-Ala-Leu-Ala-
Ala-Arg-Asp-Phe-Ile-Ala-Trp-Leu-Ile-Ala-Thr-Lys-Ile-Thr-Asp-Z2-R2
 wherein:
R¹ is hydrogen, C₁₋₄ alkyl (e.g. methyl), acetyl, formyl, benzoyl or trifluoroacetyl
X5 is Ser or Thr
X11 is Ala or Ser
R² is NH₂ or OH;
Z¹ and Z² are independently absent or a peptide sequence of 1-6 amino acid units of Lys; or a pharmaceutically acceptable salt or derivative thereof;
for providing an liquid pharmaceutical formulation which is stable for 24 months when stored at 2-8°C, wherein the formulation comprises:
(a) the GLP-2 analogue at a concentration of about 2 mg/mL to about 30 mg/mL;
(b) a buffer selected from the group consisting of a histidine buffer, mesylate buffer and acetate buffer, the buffer being present at a concentration of about 5 mM to about 50 mM;
(c) a non-ionic tonicity modifier selected from the group consisting of mannitol, sucrose, glycerol and sorbitol at a concentration of about 90 mM to about 360 mM; and
(d) arginine q.s. to provide a formulation having a pH of about 6.6 to about 7.4;
wherein the formulation contains 5% or less of the GLP-2 analogue in the form of covalently bonded oligomeric products.

54. The use according to claim 53, wherein formation of covalently linked oligomers of the GLP-2 analogue is inversely dependent on the concentration of the GLP-2 analogue in the formulation.

55. The use according to claim 53 or claim 54, wherein the total acetate concentration arising from the GLP2 analogue in the formulation is less than or equal to 11% acetate per mg GLP-2 analogue.

56. The use according to any one of claims 53 to 54, wherein the formulation has a viscosity between 0.8 and 2.0 mPa/sec measured at 25°C.
